# EUROPEAN PATENT SPECIFICATION

(11) **EP 1 610 720 B1**
(45) Date of publication and mention of the grant of the patent: **18.02.2009**
(21) Application number: 04758384.4
(22) Date of filing: 26.03.2004
(51) Int. Cl.: A61F 2/04, A61F 5/00

(54) **ANTI-OBESITY DEVICES**
VORRICHTUNGEN GEGEN FETTLEIBIGKEIT
DISPOSITIFS ANTI-OBESITE

(30) Priority: 28.03.2003 US 459060 P; 16.05.2003 US 471413 P
(43) Date of publication of application: 04.01.2006
(73) Proprietor: GI Dynamics, Inc., Lexington MA 02421 (US)
(72) Inventor: LEVINE, Andy, H., Newton, MA 02461 (US); CVINAR, John, F., Winchester, MA 01890 (US); MEADE, John, C., Mendon, MA 01756 (US)
(74) Representative: Jenkins, Peter David
(86) International application number: PCT/US2004/009269
(87) International publication number: WO 2004/087014

(56) References cited:
- WO-A-00/12027
- WO-A-01/45485
- US-A- 5 306 300
- US-A- 5 314 473
- US-A1- 2001 020 190
- US-A1- 2003 040 804
- US-A1- 2003 040 808
- PATENT ABSTRACTS OF JAPAN vol. 016, no. 552 (C-1006), 20 November 1992 (1992-11-20) -& JP 04 212348 A (OLYMPUS OPTICAL CO LTD), 3 August 1992 (1992-08-03)

## Description

### BACKGROUND OF THE INVENTION

According to the Center for Disease Control(CDC), over sixty percent of the United States population is overweight, and almost twenty percent are obese. This translates into 38.8 million adults in the United States with a Body Mass Index (BMI) of 30 or above. The BMI is defined as a person's weight (in kilograms) divided by height (in meters), squared. To be considered clinically, morbidly obese, one must meet at least one of three criteria :(i)BMI over 35 ; (ii) 45 kg (100 pounds) overweight; or (iii) 100% above an "ideal" body weight. There is also a category for the super-obese for those weighing over 159 kg (350 pounds).

Obesity is an overwhelming health problem. Because of the enormous strain associated with carrying this excess weight, organs are affected, as are the nervous and circulatory systems. In 2000, the National Institute of Diabetes, Digestive and Kidney Diseases(NIDDK) estimated that there were 280,000 deaths directly related to obesity. The NIDDK further estimated that the direct cost of healthcare in the U. S. associated with obesity is $51 billion. In addition, Americans spend $33 billion per year on weight loss products. In spite of this economic cost and consumer commitment, the prevalence of obesity continues to rise at alarming rates. From 1991 to 2000, obesity in the U. S. grew by 61%. Not exclusively a U.S. problem, worldwide obesity ranges are also increasing dramatically.

One of the principle costs to the healthcare system stems from the co-morbidities associated with obesity. Type-2 diabetes has climbed to 7.3% of the population. Of those persons with Type-2 diabetes, almost half are clinically obese, and two thirds are approaching obese. Other co-morbidities include hypertension, coronary artery disease, hypercholesteremia, sleep apnea, and pulmonary hypertension.

Although the physiology and psychology of obesity are complex, the medical consensus is that the cause is quite simple -- an over intake of calories combined with a reduction in energy expenditures seen in modem society. While the treatment seems quite intuitive, the institution of a cure is a complex issue that has so far vexed the best efforts of medical science. Dieting is not an adequate long-term solution for most people. Once an individual has slipped past the BMI of 30, significant changes in lifestyle are the only solution.

There have been many attempts in the past to surgically modify patients' anatomies to attack the consumption problem by reducing the desire or ability to eat. Stomach staples, or gastroplasties, to reduce the volumetric size of the stomach, therein achieving faster satiety, were performed in the 1980's and early 1990's. Although able to achieve early weight loss, sustained reduction was not obtained. The reasons are not all known, but are believed related to several factors. One of which is that the stomach stretches over time increasing volume, while psychological drivers motivate patients to find creative approaches to literally eat around the smaller pouch.

There are currently two surgical procedures that successfully produce long-term weight loss: the Roux-en-Y gastric bypass; and the biliopancreatic diversion with duodenal switch (BPD). Both procedures reduce the size of the stomach plus shorten the effective-length of intestine available for nutrient absorption. Reduction of the stomach size reduces stomach capacity and the ability of the patient to take in food. Bypassing the duodenum makes it more difficult to digest fats, high sugar, and carbohydrate rich foods. One objective of the surgery is to provide feedback to the patient by producing a dumping syndrome if they do eat these food products. Dumping occurs when carbohydrates directly enter the jejunum without being first conditioned in the duodenum. The result is that a large quantity of fluid is discharged into the food from the intestinal lining. The total effect makes the patient feel light-headed and results in severe diarrhea. For reasons that have not been determined the procedure also has an immediate therapeutic effect on diabetes.

Although the physiology seems simple, the exact mechanism of action in these procedures is not understood. Current theory is that negative feedback is provided from both regurgitation into the esophagus and dumping when large volumes of the wrong foods are eaten. Eventually, patients learn that to avoid both these issues they must be compliant with the dietary restrictions imposed by their modified anatomy. In the BPD procedure, large lengths of jejunum are bypassed resulting in malabsorption and therefore, reduced caloric uptake. In fact, the stomach is not reduced in size as much in the BPD procedure so that the patient is able to consume sufficient quantities of food to compensate for the reduced absorption. This procedure is reserved for the most morbidly obese as there are several serious side effects of prolonged malabsorption.

Unfortunately, these procedures carry a heavy toll. The morbidity rate for surgical procedures is alarmingly high with 11% requiring surgical intervention for correction. Early small bowel obstruction occurs at a rate of between 2 to 6% in these surgeries and mortality rates are reported to be approximately 0.5 to 1.5%. While surgery seems to be an effective answer, the current invasive procedures are not acceptable with these complication rates. Laparoseopic techniques applied to these surgeries provide faster recovery but continue to expose these very ill patients to high operative risk in addition to requiring an enormous level of skill by the surgeon. Devices to reduce absorption in the small intestines have been proposed (See U. S. Patent Number 5,820, 584 (Crabb), U. S. Patent Number 5,306, 300 (Berry) and U. S. Patent Number 4,315, 509 (Smit)). However, these devices have not been successfully implemented.

US-A-2003/040808 and US-A-2003/0408804 disclose gastrointestinal implant devices.

### SUMMARY OF THE INVENTION

The present invention provides a gastrointestinal implant device according to claim 1.

In general, the present invention is related to means to promote weight loss in humans. More specifically, the invention relates to a restrictive device implanted by a physician in the upper part of the stomach. The restrictive device can be two- piece device including anchoring cuff and a removable member with an aperture through which food transits. First, the restrictive device divides the stomach into two chambers: an upper chamber, near the esophagus, and a lower chamber. Dividing the interior of the stomach in this manner restricts the volume of the upper stomach available for the uptake of food. Thus, the size of the stomach immediately available for food is effectively reduced in a minimally invasive manner. Further, the restrictive device includes an aperture of a selectable size through which food can pass. Thus, the device also limits the rate at which food is passed from the esophagus to the upper portion of the stomach and into the intestine. Being removable, the invention also permits the physician to change the size of the opening in a minimally invasive manner by replacing the member with another member having an aperture of a different size and/or shape.

Additionally, the invention relates to a means to endoscopically restrict stomach capacity and selectively bypass the stomach, or a portion of the intestine, or a combination of bypassing both the stomach and a portion of the intestine. The restrictive device, as described above, can be combined with an elongated tube, or sleeve. Such a device eliminates contact of food with a selectable portion of the stomach, allowing a physician to endoscopically create an equivalent to the Roux- en-y weight loss procedure, a combination of restrictive and malabsorption procedure. The intestinal sleeve may be a chyme sleeve such as described in prior U. S. Utility Patent Application Nos. 10/339, 786 entitled, "Bariatric Sleeve", filed January 9,2003, (see US-A-7025791) and 10/726,011, entitled "Anti-Obesity Devices", filed on December 2, 2003, (see US-A-2003/027548). The intestinal sleeve may also be an enzyme sleeve such as described in prior U. S. Provisional Patent Application No. 60/459,060, entitled, "Enzyme Sleeve, "filed March 28, 2003, (see US-A-2004/249362).

The sleeve is attached to the restrictive device thereby eliminating contact of food with a selectable portion of the stomach. In some embodiments, the sleeve is further anchored to a portion of the gastrointestinal tract, such as at or near the pylorus, to keep the lower end of the tube from moving into the stomach or down into the intestines. Two embodiments are described. One is a continuous piece: restrictive device, stomach sleeve, sleeve anchor and intestine sleeve. The second is a two part system: first, a restrictive device, stomach sleeve and anchor-connector, and second, a pylorus anchor and intestine sleeve.

By anchoring the sleeve device in the upper part of the stomach, it becomes both a restrictive device and an intestinal sleeve. One is then able to endoscopically create a reversible and adjustable weight loss procedure similar to the Roux-en-y, a combination of restrictive and malabsorption procedure.

A gastrointestinal implant device includes a restrictive member and an anchor. The restrictive member can be a membrane, and is configured for implantation into an upper portion of an animal's stomach. When implanted, the restrictive membrane divides the inner volume of the stomach into a proximal chamber and a distal chamber. The anchor is fixedly coupled to the stomach. The restrictive membrane, however, is removably coupled to the anchor, thereby securing the restrictive membrane within the stomach.

The restrictive membrane is planar, and includes an exterior perimeter adapted to contact inner walls of the stomach. For example, the restrictive membrane can be substantially circular having an external diameter between 7 and 20 centimeters. The restrictive membrane also defines an interior aperture. The interior aperture can also be substantially circular, having a diameter, for example, between about 1 and about 5 centimeters. The restrictive membrane is preferably both flexible, allowing the restrictive screen to deform, and substantially non-elastic, ensuring that the surface area of the restrictive membrane does not vary substantially when acted upon by stresses present within the stomach.

The restrictive membrane can be formed from a permeable material impregnated with an impermeable coating. For example, the gastrointestinal implant device can be formed of a permeable material, such as a polyester fiber (e.g., DACRON® polyester), that is coated with a material such silicone, urethane, and combinations thereof. Additionally, the restrictive membrane can be formed from a composite material prepared using an otherwise elastomeric material together with a matrix of fibers. The fibers, when impregnated in the elastomeric material, alter its properties, rendering it substantially non-elastomeric.

The anchor is configured to be fixedly coupled to the stomach; whereas, the restrictive membrane is removably coupled to the anchor. Thus, the restrictive membrane to be removed and replaced as necessary, without having to remove the anchor. To facilitate removable coupling to the anchor, the restrictive membrane, includes a feature configured for coupling. For example, the feature can be a plurality of loops that couple to a respective plurality of corresponding hooks provided on the anchor.

The anchor can be fixedly coupled to the stomach using a number of retractable spring clips, each spring clip is configured to penetrate the muscular tissue of the stomach. The anchor can also include a spring clip-retaining device configured to retain the spring clips in a non-deployed position during insertion and/or removal of the anchor. For example, the retaining device can be a retaining ring removably coupled to the anchor. The retaining ring can include a respective number of slots for engaging the number of spring clips securing the clips in a non- deployed position. Alternatively, the spring-clip-retaining device can be a feature on the anchor itself. For example, the anchor can include a retaining aperture, or slot into which the tip of a spring clip can be inserted. thereby stowing it in a non- deployed position. The anchor is generally made from an elastic material, such as a shape-memory material. For example, the shape-memory material can include a nickel-titanium (Ni-Ti) alloy, commonly referred to as Nitinol.

The implant device of the invention can be used in a process for treating obesity in an animal. The process includes providing a restrictive member and an anchor. The anchor is fixedly coupled the anchor to an upper portion of an animal's stomach. The restrictive member can be a membrane, and is removably coupled to the anchor, the restrictive membrane.

The restrictive device can also be combined with a removable, variable length elongated tube. The elongated tube is open at both ends, and coupled at its proximal end to the variable restrictive device. The elongated tube extends at least near the pylorus and is adapted to limit absorption of nutrients in the stomach. The device also includes an anchor coupled to the elongated tube for securing at least a distal portion of the tube to the gastrointestinal tract.

In some embodiments, the elongated tube includes a flexible sleeve, that can also be a non-supported. For example, the flexible sleeve can be formed of a material including polytetrafluoroethylene (PTFE), expanded PTFE, Fluorinated. Ethylene Polymer (FEP), polypropylene, polyethylene, and combinations thereof. Preferably, the flexible tube is formed of a material having a coefficient of friction of less than about 0.2. The flexible sleeve can include a base material and a coating, such as a silicone-based coating, a polyurethane-based coating, and combinations thereof.

The elongated tube can be formed with variable lengths, that can extend into the intestine. The anchor secures the elongated tube within the gastrointestinal tract. For example, the anchor can secure the elongated tube to a portion of the gastrointestinal tract, such as at or near the pylorus.

Additionally, the gastrointestinal implant device can include a second, elongated extension tube. The extension tube is also open at both ends, having a proximal end substantially aligned with a distal end of the elongated tube. The extension tube can be coupled to the elongated tube using a connector coupled between the proximal end of the extension tube and the distal end of the elongated tube. For example, the connector can include a hook-and-loop connector. Thus, a number of hooks on at least one end of one of the elongated tubes, hook through respective loops on a mating end of the other of the elongated tubes.

The anchor, in turn, can be collapsible. For example, the anchor can be formed froma shape memory material, such as Nitinol. Additionally, the anchor can be tubular including a number of barbs extending from the exterior surface of the anchor. The barbs are generally configured for securing the anchor to muscular tissue of the gastrointestinal tract. In some embodiments, the barbs are substantially bi-directional, extending outward, in opposing directions that are substantially parallel to the central axis of the tubular anchor.

The implant device of the invention can be used in to a process for treating obesity in an animal. The process includes the steps of endoscopically placing a removable, variable restrictive device in an upper part of a stomach, endoscopically placing a removable, variable length sleeve in the duodenum and jejunum ; and connecting the sleeve to the restrictive device. The removable, variable restrictive device can be placed by providing a restrictive membrane and an anchor, and fixedly coupling the anchor to an upper portion of an animal's stomach. The restrictive membrane is then removably coupled to the anchor. When so coupled, the restrictive membrane divides the inner volume of the stomach into a proximal chamber and a distal chamber and limits the rate that food can pass therethrough.

### BRIEF DESCRIPTION OF THE DRAWINGS

The foregoing and other objects, features and advantages of the invention will be apparent from the following more particular description of preferred embodiments of the invention, as illustrated in the accompanying drawings in which like reference characters refer to the same parts throughout the different views. The drawings are not necessarily to scale, emphasis instead being placed upon illustrating the principles of the invention.
FIG. 1 is a sectional view of a portion of the gastrointestinal tract of an animal body;
FIG. 2 is a sectional view of a portion of the stomach including one embodiment of a restrictive drum assembly;
FIG. 3A is a perspective view of the anchoring ring shown in FIG. 2;
FIG. 3B is a perspective view of the restrictive drum shown in FIG. 2;
FIG. 4 is a perspective view of a catheter containing an anchoring ring folded for delivery;
FIG. 5 is a sectional view of a portion of the stomach illustrating deployment of the restrictive drum assembly shown in FIG. 2;
FIGS. 6A and 6B are a perspective views of a portion of the restrictive drum assembly in more detail respectively illustrating the anchors in the pre-released position, and released position;
FIG. 7 is a more detailed perspective view of the anchor release ring shown in FIGS. 5, 6A, and 6B;
FIG. 8 is a perspective view illustrating in more detail the anchoring ring engaging the drum member;
FIG. 9 is a cross sectional view along A-A of FIG. 4, illustrating an alternative embodiment for securing anchors of the anchor ring in an undeployed position for endoscopic delivery and/or removal through a catheter;
FIGS. 10A and 10B are a perspective views of a release device for releasing the tips of the spring clips from the slots after the anchoring ring is in position, respectively showing a stripper bar retracted and extended;
FIG. 11 is a perspective view illustrating the jaws of the release device shown in FIGS. 10A and 10B positioned to remove the tips of the spring clips from the anchor retaining slots.
FIGS. 12A and 12B are a perspective views of a portion of the release device and the anchoring ring, illustrating the spring clips after being released, respectively showing the stripper bar in the retracted and extended positions;
FIG. 13 is a sectional view of a portion of the stomach including one embodiment of a restrictive drum assembly-sleeve combination with a one-piece sleeve extending through the stomach and into the intestine;
FIG. 14 is a perspective view of the restrictive drum assembly with a one-piece sleeve combination, showing in more detail the anchor of FIG. 13;
FIGS. 15A and 15B are schematic diagrams illustrating a side projection view of one embodiment of a tubular anchor respectively shown in an expanded configuration and in a contracted configuration;
FIG. 16A provides top and side orthonormal projection views of an alternative embodiment of a tubular anchor;
FIG. 16B is a perspective view of the tubular anchor shown in FIG. 16A;
FIG. 17 is a perspective view of a restrictive drum with an attached stomach sleeve and an attached anchoring connector;
FIG. 18 is a sectional view of a portion of the stomach showing the anatomical positioning of a restrictive drum assembly-sleeve combination with a two-piece sleeve extending through the stomach and into the intestine;
FIG. 19 is a side view of one embodiment of a two piece anchoring connector, the two pieces shown coupled together;
FIG. 20 is a side view showing in more detail the stomach sleeve connectors with locking details;
FIG. 21 is a side view of a stomach sleeve connector, showing in more detail the stomach sleeve connector locked to the anchoring stent; and
FIG. 22 is a sectional view of a portion of the stomach showing the anatomical positioning of a restrictive drum assembly-sleeve combination with an intestinal sleeve

### DETAILED DESCRIPTION OF THE INVENTION

A description of preferred embodiments of the invention follows.

One means of reducing caloric intake is to reduce the size of the stomach immediately available for the uptake of food. This can be accomplished, at least in part, with a restrictive device configured for implantation in the upper part of the stomach. The restrictive device selectively divides the stomach into two chambers, reducing the size of the stomach immediately available for the uptake of food. The restrictive device also includes an aperture, limiting the rate at which food can pass from the esophagus portion of the stomach into the lower portion of the gastrointestinal tract. Being removable, the device permitsa physician to change the size of the opening in a minimally invasive manner by replacing a removable member with another member having an aperture of a different size and/or shape.

Another means of reducing caloric uptake is to reduce the area of the gastrointestinal tract available for absorbing nutrients. Thus, the restrictive device can be combined with an elongated tube, or sleeve to selectively bypass the stomach, a portion of the intestine, or a combination of bypassing both the stomach and a portion of the intestine, allowing a physician to endoscopically create an equivalent to the Roux-en-y weight loss procedure.

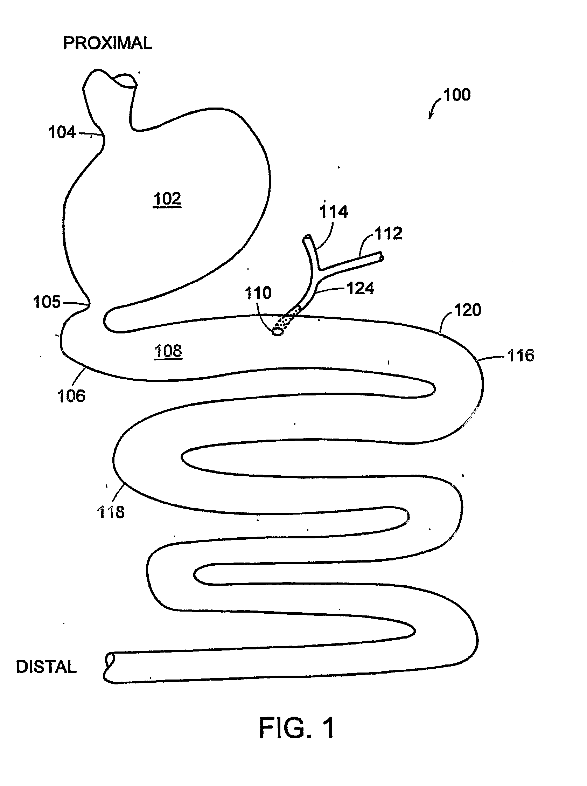

FIG. 1 is a section view of a portion of the digestive tract 100 of an animal body. Food to be digested enters the stomach 102 through the cardiac orifice 104 from the esophagus. Chyme, a semi-fluid, homogeneous creamy or gruel-like material produced by gastric digestion in the stomach exits the stomach through the pyloric orifice (pylorus) 105 and enters the small intestine 106. The pylorus 105 is a distal aperture of the stomach 102 surrounded by a strong band of circular muscle. The small intestine 106 is an average human body is a convoluted tube, about 4.6 m (15 feet) in length, extending from the pylorus 105 to the ileo-caecal valve where it terminates in the large intestine (not shown). Generally, the small intestine 106 includes three sections: 91) the duodenum 108; (ii) the jejunum 118; and (iii) the ileum (now shown they are continuous and should be noted). The first 30cm (twelve-inch) section of the small intestine 106, the duodenum 108, is the shortest, widest and most fixed part of the small intestine 106.

The duodenum 108, in turn, includes four sections: (i) superior; (ii) descending; (iii) transverse ; and (iv) ascending, which typically form a U-shape. The superior section is about 5 cm (two inches long) and ends at the neck of the gall bladder (not shown). The descending section is about 7.5 to 10 cm (three to four inches) long and includes a nipple-shaped structure, referred to as the papilla of vater 110 through which pancreatic juice from the pancreas and bile produced by the liver and stored by the gall bladder, enter the duodenum. The pancreatic juice flows from the pancreas to the papilla of vater 110, through the pancreatic duct 112. Similarly, bile flows from the gall bladder to the papilla of vater 110, through the bile duct 114. Both ducts 112,114 combine, as illustrated, before the papilla of vater 110, the anatomy of which is described in more detail below. The pancreatic juice contains enzymes essential to protein digestion; whereas, bile dissolves the products of fat digestion. Finally, the ascending section is about 5 cm (two inches) long and forms the duodenal jejunal flexure 116 where it joins the jejunum 118, the next section of the small intestine. The duodenal jejunal flexure 116 is fixed to the ligament of Treitz 120(musculus supensiollus duodeni). Thus, the juices naturally secreted into the duodenum further break down the partially digested food into particles small enough to be absorbed by the body. The digestive system 100 is described in numerous texts, such as Gray's Anatomy ("Anatomy of the Human Body,"by Henry Gray), and "Human Physiology", Vander, 3rd Ed., McGraw Hill, 1980.

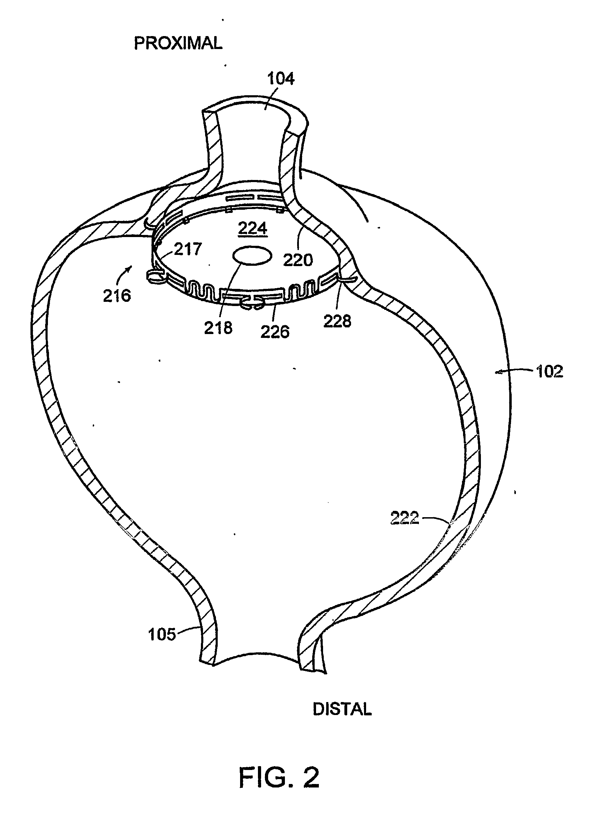

A restrictive device 216 is shown in FIG. 2, as it would sit in an expanded stomach 102. The restrictive device 216 sits in the upper portion of the stomach 102, dividing the stomach 102 into an upper chamber 220 and a lower chamber 222. Preferably, the restrictive device 216 is placed near the proximal end of the stomach 102 (near the esophagus 104) thereby defining a small, upper stomach pouch 220. For example, the restrictive device 216 can be positioned within the stomach to provide an upper stomach pouch 220 having a volume between about 30 and about 100 cubic centimeters (cc). Ingested food initially enters through the esophagus 104 and fills up the small upper stomach pouch 220, being separated by the restrictive device 216 from the remaining lower portion of the stomach 222.

The restrictive device 216 is generally planar, having an exterior perimeter 217 and defining an interior aperture 218. The restrictive device 216 is attached along its exterior perimeter 217 to the walls of the stomach 102. Ingested food retained within the upper stomach pouch 220 passes from one side of the restrictive device 216 to the other, through the aperture 218.

According to the invention the restrictive device 216 is formed as an assembly having a fixed, anchoring member, and a removable, restrictive member. For example, the restrictive device 216 includes an anchoring ring 226 and a restrictive membrane 224. The anchor ring 226 is configured to secure the restrictive device 216, along its external perimeter to the interior walls of the stomach 102. According to the invention the anchor ring 226 is permanently attached to the stomach 102, whereas the restrictive membrane 224 is removably coupled to the anchor ring 226. Thus, the restrictive membrane 224 can be implanted and removed, as required. Additionally, restrictive membranes 224 having variable apertures can be inserted and replaced, as required. For example, a restrictive membrane 224 having a large aperture 218 may be inserted at first, to allow the patient to become accustomed to the implant, and/or to provide time for the ingrowth of tissue around the anchor. Later, the restrictive membrane 224 can be removed and replaced with a different membrane 224 having a smaller aperture 218. Should the smaller aperture 218 be too small, causing difficulties for the patient, the restrictive membrane 224 can be removed and replaced again with another membrane 224, the procedure being repeated as necessary.

The anchoring ring 226 can be secured to the stomach 102 using sutures, surgical staples, and/or an adhesive, or any other suitable means of attachment. Preferably, the anchoring ring 226 is configured to promote tissue growth, thereby further securing the anchoring ring 226 in place. Alternatively or in addition, the anchoring ring 226 can be secured to the stomach 102 using retractable staples 228. The retractable staples 228 are configured to penetrate through the mucous lining and into the tissue of the stomach 102 for secure attachment to the stomach 102. As food fills the upper stomach pouch 220, considerable pressure can result along the upper surface of the restrictive membrane 224. For an adult human, the outer diameter of the membrane can range from about 7 to about 20 centimeters (cm), depending on the size of the stomach pouch desired.

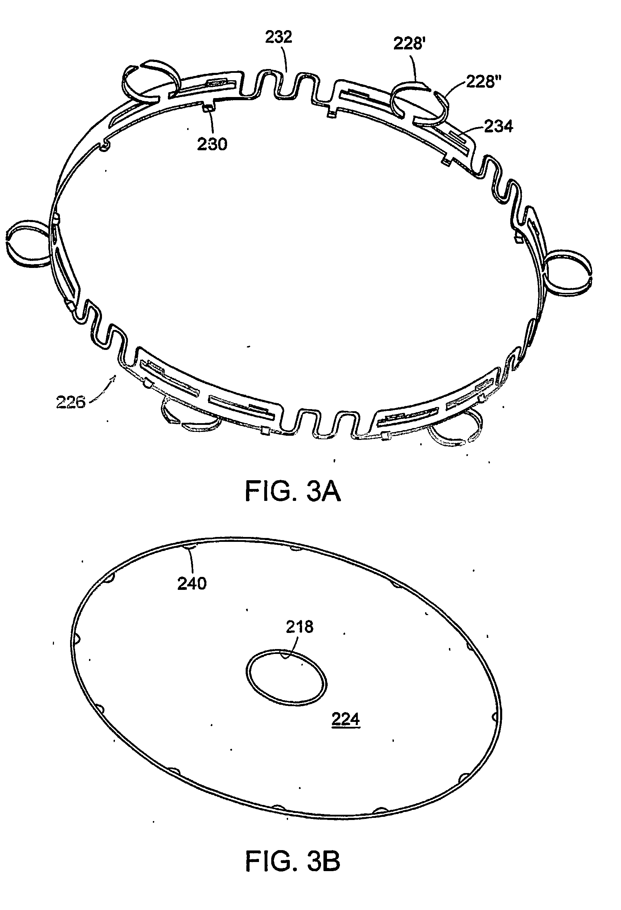

FIG. 3A shows the anchoring ring 226 in more detail with the retractable anchors 228 extended in a fully engaged position for anchoring. The retractable anchors 228 are generally attached at one end to the anchoring ring 226, the other end expending radially outward from the center of the anchoring ring 226, to engage the tissue of the stomach 102. In some embodiments, as shown, two anchors 228', 228" can be aligned in opposition, such that the extended ends of the anchors 228', 228" approach each other, being capable of grasping, or pinching the tissue of the stomach 102. The anchors 228 can be formed from cut outs in the anchoring ring 226 itself. Further, the anchors 228 can be formed of a resilient material such that they spring outward from the anchoring ring 226, when inserted, to grasp the stomach 102. Additionally, the anchors 228 can be formed from a shape memory material, such as a nickel-titanium (Ni-Ti) alloy, commonly referred to as Nitinol. The Nitinol anchors 228 can be heat formed into the position shown so that when released from the anchor retainer slots, they spring into this position.

The anchoring ring 226 is generally made of a flexible material to facilitate its delivery into the stomach, and to permit collapse of the stomach around it. For example, the anchoring ring 226 can be formed using flexible metals, polymers, or combinations thereof. Additionally, the anchoring ring 226 can include a fibrous material. In some embodiments, the entire anchoring ring 226 is made from a shape memory material, such as Nitinol.

In order to increase flexibility of the anchoring ring 226, cutouts 232 in the ring can be provided. As an additional benefit, such cut outs 232 also promote the ingrowth of tissue. Drum engagement hooks are located around the ring to engage closed slots in the removable drum membrane and hold it in place. The anchoring ring will become covered in tissue over time and is thus not removable.

FIG. 3B shows one embodiment of a restrictive membrane 224. Preferably, the restrictive membrane 224 is made from a flexible material that is substantially non-elastic. Thus, as with the anchoring ring 226, the restrictive membrane 224 is also deformable to facilitate insertion into the stomach and to permit collapse of the stomach around it. However, the restrictive membrane 224 preferably does not stretch. Such a limitation on the maximum size of the aperture 218 provides better control on the rate at which food passes from the upper stomach pouch 222 into the distal portions of the digestive tract. By providing a control on the maximum aperture size of a given restrictive membrane 224, other drum membranes having apertures with different sizes may be used, as required. The aperture 218 in the restrictive membrane 224 is selectable and may be varied between about 1 and about 5 centimeters (cm) in diameter depending on the level of restriction desired.

Exemplary material for the restrictive membrane 224 providing the desired properties include non-permeable materials, such as polymers, PTFE, ePTFE, FEP, polypropylene, polyethylene, and combinations, thereof. Alternatively or in addition, the restrictive membrane 224 can be formed from a permeable material impregnated with an impermeable coating, such as silicone or urethane. The permeable material can be a fibrous material, such as a weave. The fibers can be natural fibers or synthetic fibers. Preferably, the synthetic fibers can be a polyester fibers, such as DACRON® polyester. Additionally, otherwise elastic materials, such as silicone, urethanes, and/or polymers, can be impregnated with fibers to limit stretching, rendering them essentially non-elastomeric.

To facilitate insertion and removal, some embodiments of the drum membrane 224 include a number of closed loops, or slots 240 arranged around the periphery. These slots 240 are configured for removably coupling to corresponding engagement hooks 230 around the periphery of the anchoring ring 226.

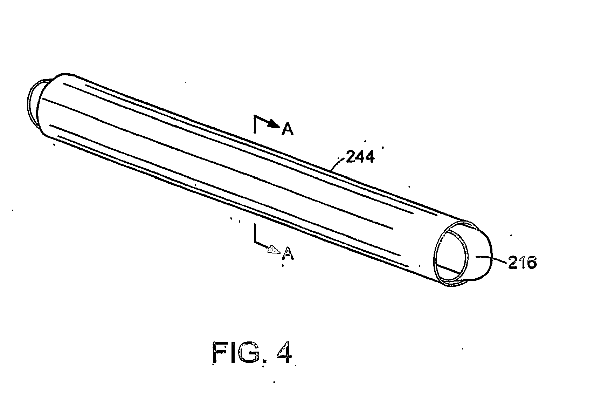

As described above, the anchoring ring 226, and the drum membrane 224 are collapsible and are shown in FIG. 4 in a collapsed state. Thus, the anchoring ring 226, and/or the restrictive membrane 224 can be folded in an introduction tube 244 for endoscopic delivery through the esophagus. In the collapsed state the anchoring ring 226, the restrictive membrane 224, and/or the entire restrictive device 216, can be collapsed having a maximum radial dimension along the tube, ranging from about 5 to about 15 millimeters (mm) in diameter. The anchoring ring 226 can be introduced through the esophagus and then released from its introduction tube 244. The anchoring ring 226 can then be aligned or pulled into place, up against the stomach 102 to seat it in the correct position using graspers (not shown).

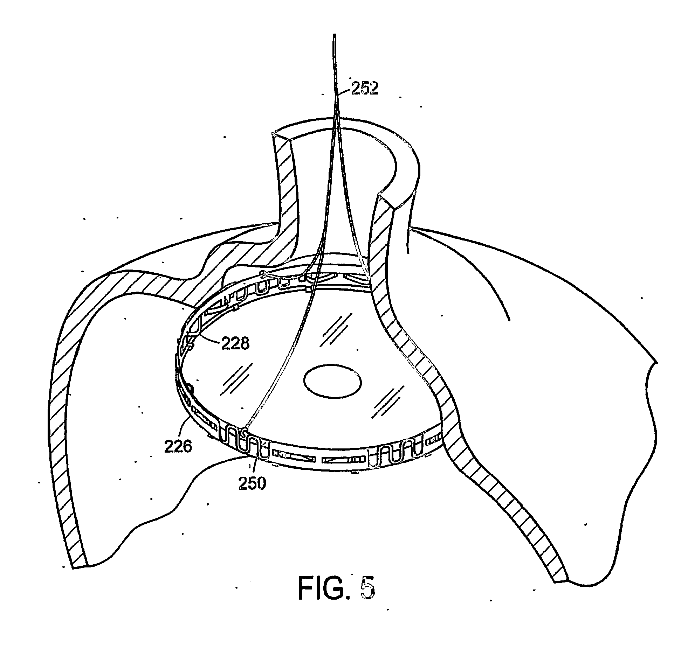

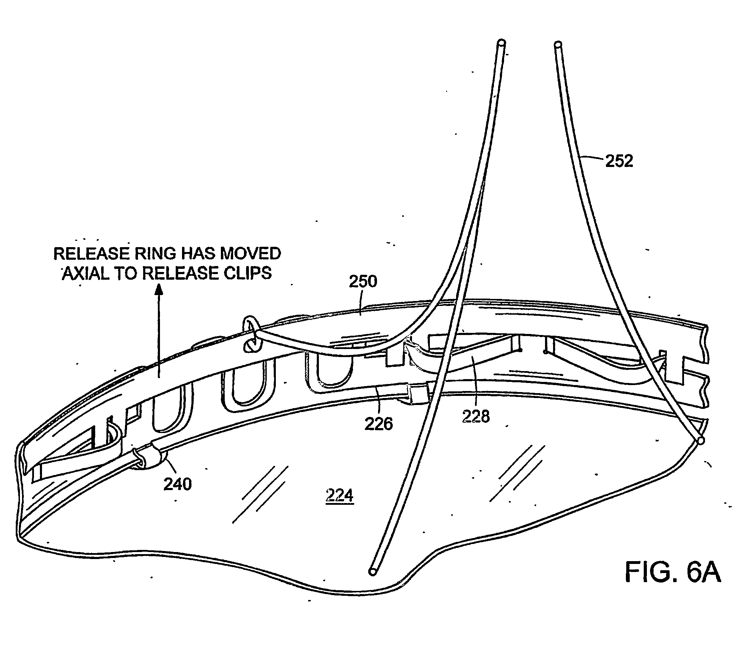

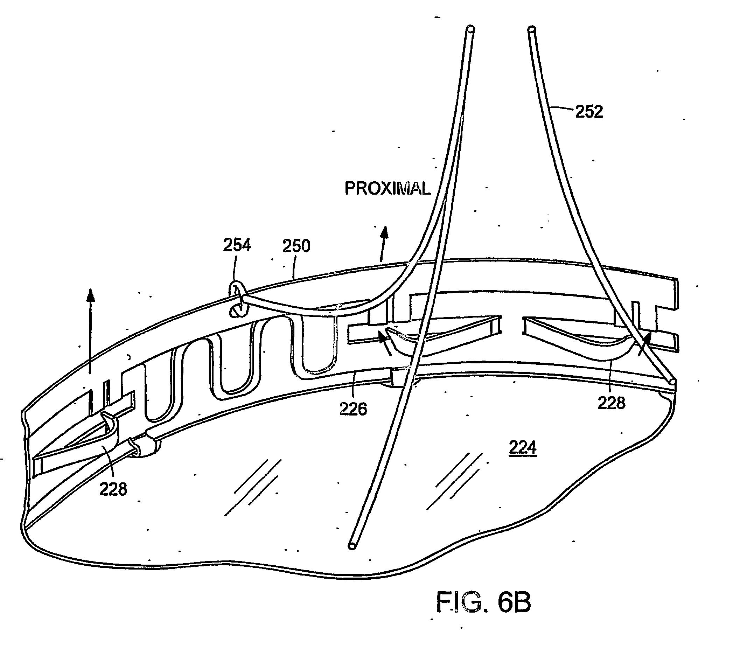

For example, referring now to FIG. 5, the anchoring ring 226 is first positioned to a desired location within the stomach 102 prior to anchoring. In some embodiments, the anchors 228 are held in their undeployed position in the respective anchor retainer slots by an anchor release ring 250. One or more wires 252 can be attached to the anchor release ring 250, and the anchor release ring coupled to the anchoring ring 226 using an interference fit. When the anchoring ring 226 is in place, the wires 252 attached to the anchor release ring 250 are pulled toward the proximal end of the stomach thereby separating the anchor release ring 250 from the anchoring ring 226 by pulling the anchor release ring 250 up and off of the anchoring ring 226. As described above, removal of the anchor release ring 250 releases the anchors 228 from their respective anchor retainer slots, allowing the anchors 228 to bend into their pre-formed position. Thus, the anchors 228 dig into the stomach muscle tissue to anchor the anchoring ring 226 into place. This is shown more clearly in FIG. 6A where the anchor release ring 250 is holding the anchors 228 back, in a stowed position for insertion and removal. In FIG. 6B, the anchor release ring 250 is removed in the direction shown, freeing the anchors 228 and allowing them to bend outward, extending into a deployed position. The anchor release ring 250 can then be pulled back using one of the wires 252 into a delivery sleeve, or catheter, for endoscopic removal from the body.

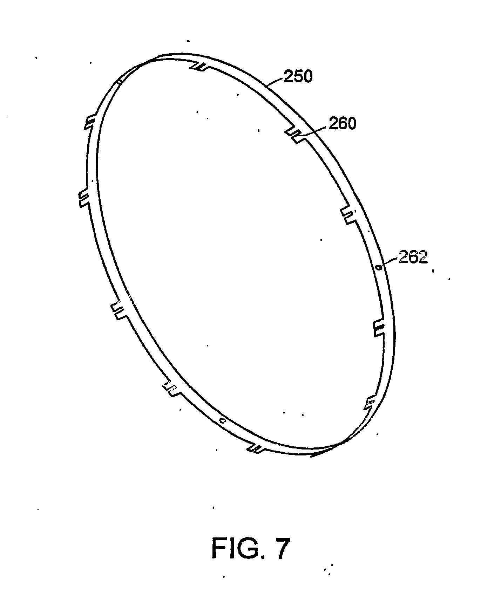

FIG. 7 shows one embodiment of an anchor release ring 250. The anchor release ring 250 includes a number of anchor retention slots 260, each slot configured, when coupled to the anchoring ring 226, to retain a corresponding anchor 228. The anchor release ring 250 can be made using any of the materials used for the anchoring ring. The anchor release ring is generally formed with a shape configured to contour to the perimeter of the anchoring ring 226. Thus, as shown, the anchoring ring 250 is circular, having a diameter substantially the same as, or slightly less than the diameter of a circular anchoring ring 226. Additionally, the anchor release ring 250 includes a feature to facilitate its insertion and removal. For example, the feature can be a hole 262 through which one end of a wire can attach.

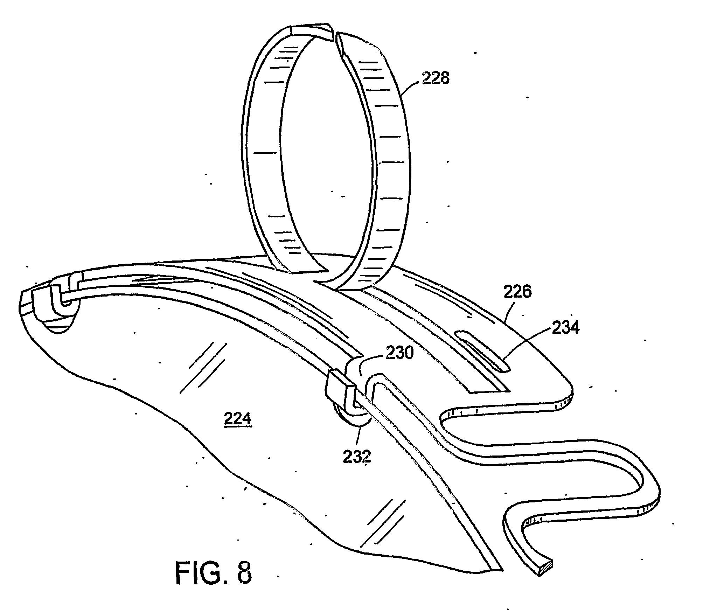

After the anchoring ring 226 is anchored by the anchors 228 and the anchor release ring 250 has been removed, the restrictive membrane 224 is endoscopically delivered using a delivery tube similar to the delivery tube shown in FIG. 4 for delivering the anchoring ring. After the delivery tube has been inserted, the drum member 224 is removed from the tube by graspers and placed in position such that each drum engagement hook 230 on the anchoring ring 226, as shown in FIG. 3A, engages a respective retainer slot 232 on the drum membrane 224, as shown in FIG. 3B. FIG. 8 illustrates the drum cover hooks 230 on the anchoring ring 226 engaged with the retainer slots 232 on the restrictive membrane 224.

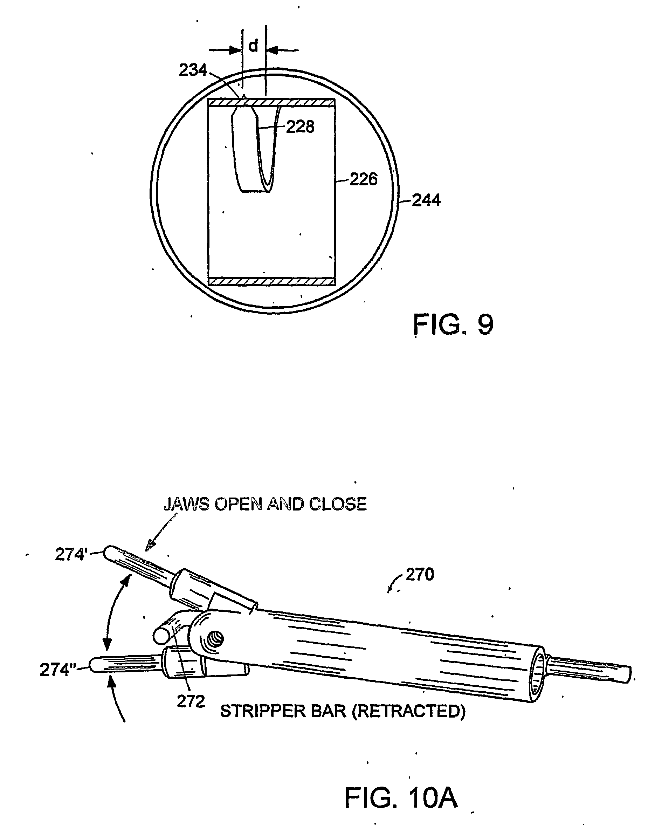

FIG. 9 illustrates an alternative embodiment for holding the anchors 208 in an undeployed position. As shown in cross-section, the anchoring ring 226 is in its collapsed position in the introducing tube 244. The tip of one of the anchors is inserted into a respective anchor retaining slot 234 in the anchoring ring 236. As shown, the slot 234 can be located such that it is out of alignment with the anchor 238 by a distance 'd.' Thus, the spring action of the anchor 238 retains the tip within the slightly misaligned slot during insertion and/or removal. The anchoring ring 226 can be introduced through the esophagus 104 and then released from its introduction tube 244. The anchoring ring 226 is then pulled with graspers up against the stomach to seat it in the correct position. Once delivered and positioned into place, the anchors 238 are removed from their respective retaining slots and allowed to spring outward, thereby grasping the tissue of the stomach as described above.

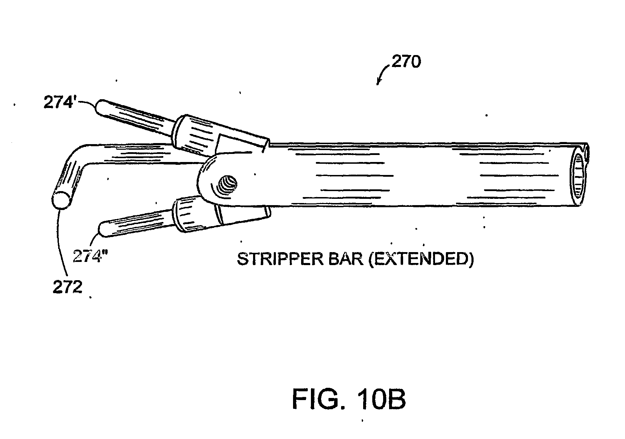

FIG. 10A illustrates one embodiment of a release device 270 configured for releasing the tips of the anchors 238, or spring clips from their respective anchor retaining slot 234 after the anchoring ring is positioned. Each spring clip 238 is individually released by the release device 270. The release device 270 is endoscopically delivered to the stomach 102. The release device 270 includes jaws 274', 274" (generally 274) that open and close, and a stripper bar 272 positioned between the jaws 274. The stripper bar 272 can be advanced and retracted along a longitudinal axis of the release device 270. FIG. 10B illustrates the device shown in FIG. 10A with the stripper bar extended. While the stripper bar 272 is in a retracted position, the jaws 274 can open and close.

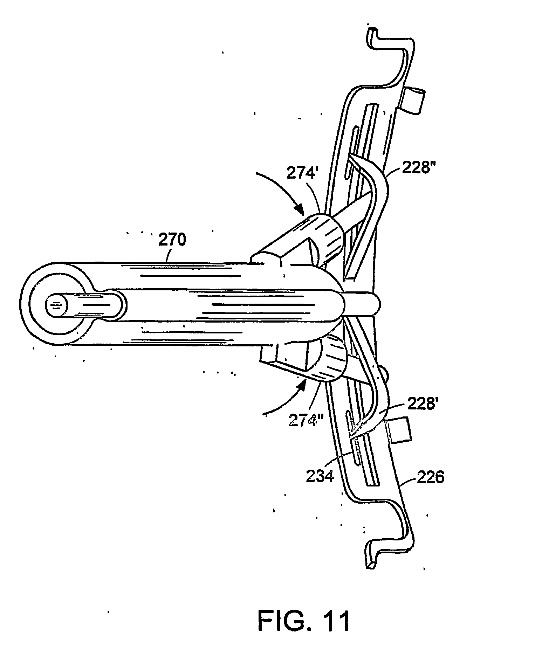

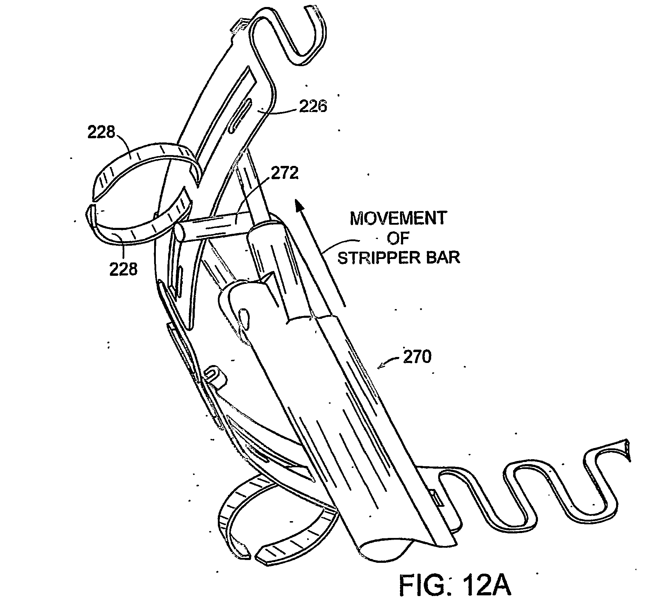

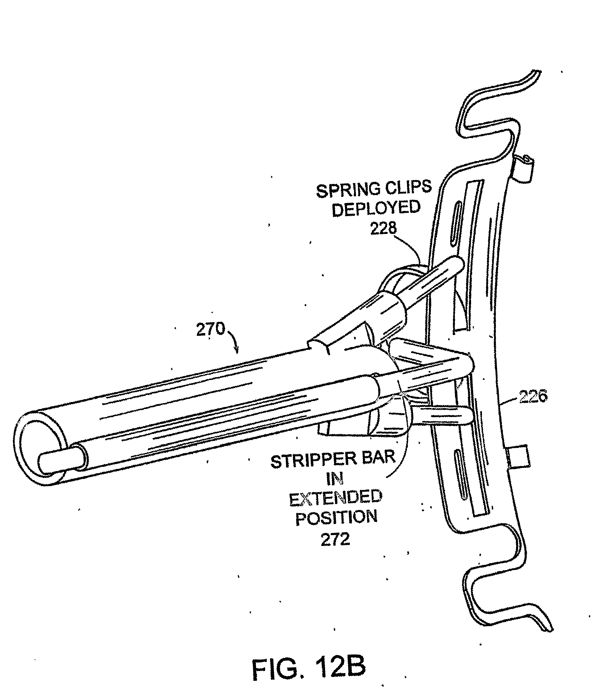

FIG. 11 illustrates the jaws 274 of the device positioned to remove the tips of two opposing spring clips 228', 228" from their respective retaining slots 234. The stripper bar 272 is initially in a retracted position. Each jaw 274 is inserted under a respective spring clip 228. As the jaws 274 are closed, the jaws 274 wedge each tip of the opposing spring clips 228', 228"out of their respective retaining slots 234. FIG. 12A illustrates the spring clips 228', 228"after release. The stripper bar 272 is moved in a longitudinal direction toward the distal end of the jaws 274. The movement of the stripper bar 272 in one action, strips the anchoring ring 226 from the jaws and releases the spring clips 228', 228". FIG. 12B illustrates the stripper bar 272 in an extended position after the spring clips 228', 228"have been deployed.

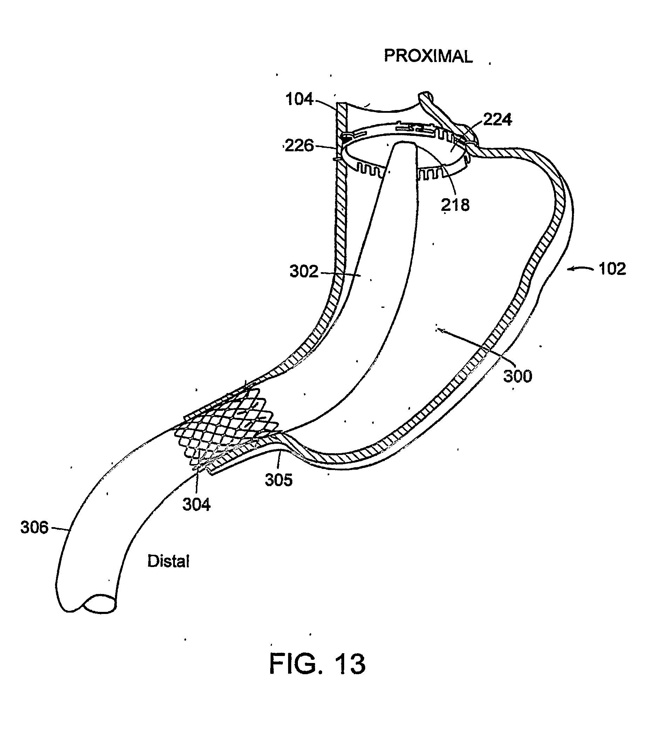

In an alternative embodiment, referring now to figure 13, the restrictive device 216 described above can be combined with a removable, elongated tube. For example, the elongated tube can be a variable length sleeve 302 coupled at its proximal end to the restrictive device 216, and anchored at its distal end to a portion of the gastrointestinal tract, such as the pylorus 305. In this manner, the sleeve 302 essentially results in a stomach bypass, providing a similar result to the Roux-en-y surgical bypass. The sleeve can be a removable, variable length sleeve as described in co-pending U. S. Utility Patent Application No. 10/339, 786 (see US-A-7025791). The removable restrictive membrane 224 allows access to the removable variable length sleeve 302, allowing the variable length sleeve 302 to be placed and removed endoscopically. With both devices removable 216,302, the level of malabsorption and restriction can be varied by varying the length of the sleeve 302 and varying the diameter of the aperture218 in the restrictive membrane 224. Further, the length of the sleeve 302 can be varied to extend beyond the pylorus 305, having a distal end 306 extending into the intestine.

The distal end of the sleeve 306 can be placed within the interior of the gastrointestinal tract 100 terminating at a predetermined location that is generally determined by the length of the sleeve 306. Typically, in human applications, the overall length of the sleeve 306 ranges from about 30 cm (one foot) to about 150 cm (five feet). In some embodiments, the device can be up to 3 m (10 feet) in length to extend into the ileum. The typical length of the sleeve 306 is about 60 cm (2 feet) extending from the anchor 304 to below the ligament of Treitz 120. The length of the sleeve 306 is generally selected to bypass the duodenum 108 and at least a portion of the jejunum 118. However, sleeves306 of various lengths can be used to adjust the amount of absorption. For example, the length of the sleeve 306 can be increased to further decrease absorption by bypassing a longer section of the jejunum 108. Additionally, the length of the sleeve 306 can be variable and dependent on the patient's Body Mass Index (BMI).

FIG. 13 shows the positioning of a one-piece sleeve assembly in the overall anatomy. The sleeve 302 can be attached at its proximal end to the restrictive membrane 224 using mechanical fasteners, chemical fasteners, such as adhesives, melting or other means. The sleeve 302 can similarly be attached to the anchoring stent 304 in the middle using any of the above attaching techniques. The anchoring stent 304 anchors the sleeve assembly 300 to a portion of the gastrointestinal tract, such as at or near the pylorus. Alternatively, the anchor 304 can secure the sleeve assembly 300 in the stomach 102, or below the pylorus 305 such as in the small intestine 120. An anchoring stent is described in co-pending U. S. Utility Patent Application No. 10/339, 786 (see US-A-702791).

The sleeve material is preferably thin and conformable so that it collapses in the intestine to a small volume to minimize bowel irritability. In some embodiments, the thin-walled sleeve 302 is naturally in a collapsed state and is opened only by pressure from chyme within the sleeve 302. Further, the sleeve material preferably has a low coefficient of friction (e. g., less than about 0.20) so that chyme slides easily through it and digestive enzymes slide easily around it. Further, the sleeve is preferably formed using a material having a low permeability to fluids, so that the chyme neither mixes with digestive enzymes, nor contacts the bowel wall over the length of the sleeve 302. Thus, as the digestive enzymes are isolated from the chyme by the sleeve, they do not significantly breakdown the chyme. Still further, the sleeve material is preferably biologically inert, impervious to digestive fluids, and non-irritating to the tissues.

In some embodiments, the sleeve material having the above-recited properties is formed using expanded polytetrafluoroethylene (ePTFE) with a wall thickness of about 0.013 cm (0.005 inch) with an internodal distance of less than about 5 microns. Notably, ePTFE is hydrophobic, yet slightly porous. The very small pores may become clogged over time. The porosity can be reduced by selectively coating the material on the inside, and/or the outside, and/or in the pores with dilute solutions of a sealant material, such as silicone or polyurethane.

In other embodiments, the sleeve 302 can be formed using a thin film of TEFLON® (e. g., PTFE, or FEP, or a combination thereof), polypropylene or polyethylene. For example, the film can have a wall thickness of less than about 0.0025 cm (0.001 inch). The sleeve material must be sufficiently thin and pliable to permit peristalsis to propel the chyme inside the tube.

As described above, the sleeve 302 is secured at its proximal end by the restrictive screen 216, which is anchored in the stomach 102. The sleeve 302 can also be secured using an anchor 304 at its distal end, or anywhere in between the restrictive screen 216 and the distal end of the sleeve 302. Thus, an anchor 304 can be first attached to the sleeve 302, then secured to a predetermined location along the gastrointestinal tract 100, anchoring the sleeve302 at that location. For example, the anchor 304 can be secured to the pylorus 305, as shown.

The anchor 304 can be formed as a collapsible and self-expanding device, such as a collapsible, self-expanding tube, or stent. Thus, the anchor 304 can be securely attached to the gastrointestinal tract 100 using an interference fit alone or in combination with one or more anchoring barbs configured to penetrate the muscular tissue of the gastrointestinal tract100, as described in co-pending U. S. Patent Application Nos. 10/339,786, (see US-A-7025791), 10/726, 011 (see US-A-2007/027548), and as also described in co- pending U. S. Provisional Application Nos. 60/528,084, and 60/544, 527, entitled "Methods And Apparatus For Using A Sleeve Within The Gastrointestinal Tract", filed on February 13,2003, (for both see US-A-2005/0125020). Alternatively, or in addition, the anchor 304 can be attached to the gastrointestinal tract 100 using sutures, surgical staples, an adhesive, a combination of these, or any other suitable means. Preferably, the anchor 304 is removably attached, such that the device 300 can be implanted and removed as required with relative ease. For example, the anchor 304 can be formed from a shaped memory material, such as Nitinol. The Nitinol anchor 304 can be similar in design to the pyloric anchoring device described in co-pending U. S. Patent Application Serial Numbers 10/339,786, entitled "Bariatric Sleeve", filed January 9,2003 (see US-A-7025791), and 10/726,011, entitled "Anti-Obesity Devices", filed December 2,2003, (see US-A-2007/027548).

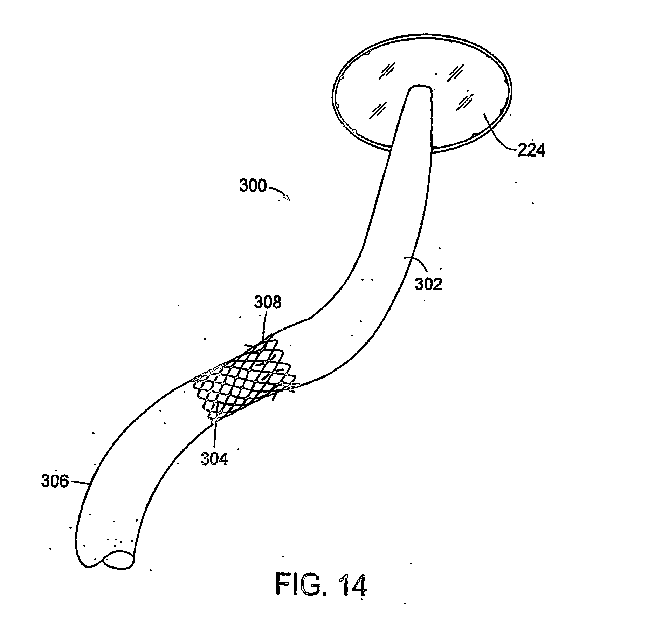

FIG. 14 shows a one-piece sleeve 300 attached to the restrictive drum plate 224. This assembly is collapsed and endoscopically introduced into the stomach. The distal end of the sleeve 300 is inserted into the duodenum with the attached anchoring stent 304 being inserted into the pylorus 305. The stent 304 includes a plurality of opposed barbs 308 for anchoring the implant device to the muscular pylorus 305 in the stomach 102. The diameter of the stent 304 is dependent on the diameter of the pyloric orifice305, which is between about 20 mm (0.8 inch) and about 28 mm (1.1 inch), based on human anatomy variations. In one embodiment, the length 'L' of the stent is selected to extend through the pylorus 305 and keep the pylorus 305 permanently open to induce "dumping syndrome. "In an alternate embodiment, a stent 304 with a shorter length 'L' allows the pylorus 305 to open and close normally. The anchoring stent 304 is self expanding and anchors in the wall of the pylorus 305. The anchoring stent 304 is placed in the upper portion of the stomach, again via endoscopic means as described above. With the anchor ring 226 in place, the drum restrictor plate 224 with the attached upper portion of the sleeve 302 is coupled to the anchor ring 226.

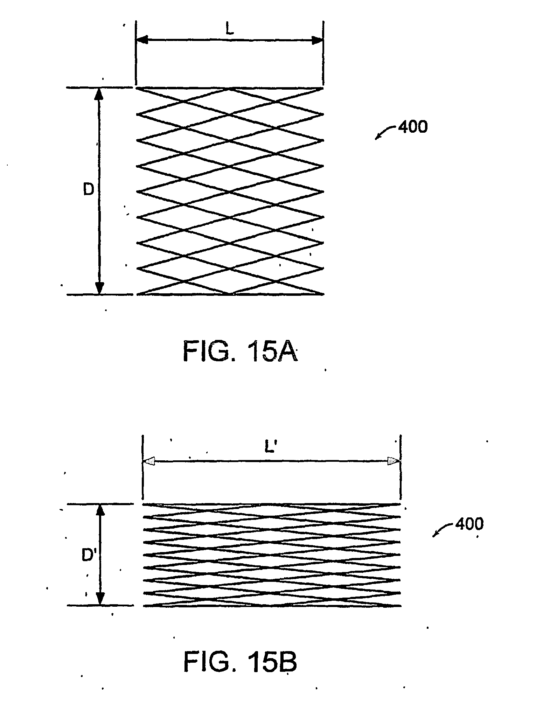

An exemplary tubular anchor 400 can be formed using a network of struts. Referring to FIG. 15A, an exemplary anchor 400 formed in this manner is shown in an expanded configuration. The same anchor 400 shown in a collapsed configuration is illustrated in FIG.15B. Thus, the anchor 400 can be formed from interconnnecting struts that form a mesh (e. g., a network of struts). For example, the struts can form a mesh having diamond spaced openings, as illustrated, that are sufficiently flexible to allow the stent to be collapsed inside a delivery catheter and have sufficient elasticity to expand to secure the anchor to the gastrointestinal tract 100 once the catheter is withdrawn. As described above, the struts can be formed from a shape memory material, such as Nitinol. Thus, in some embodiments, the anchor is compliant. Alternatively, the anchor can be constructed of a rigid material, such as a rigid metal or plastic. The rigid material can be expandable to facilitate insertion into the body, but once expanded, can retain its expanded shape. In some embodiments, the anchor 400 is non-expandable.

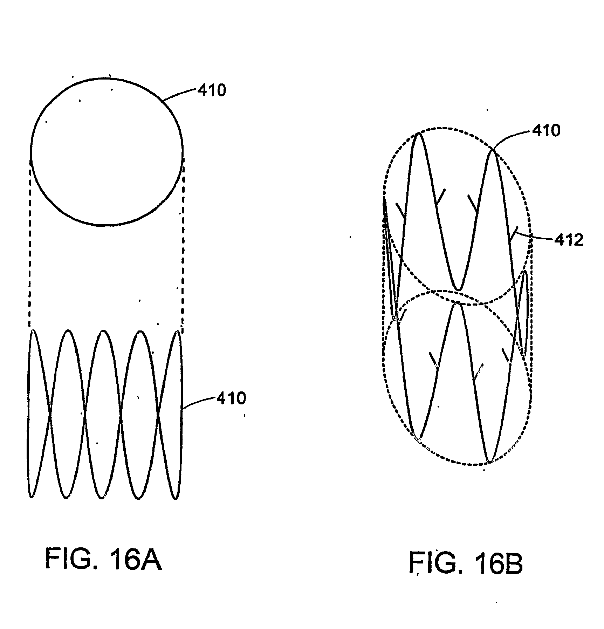

The tubular anchor 304 can also be formed using a single, continuous supporting member, such as a wire having the "wave" shape illustrated in FIGS. 16A and 16B. The wave shape of the supporting member allows for compression in the radial direction to facilitate insertion and/or removal. As shown in FIG. 16B, the wave anchor 410 can optionally include one or more barbs 412 configured for securing the anchor in its installed position. For example, the wave anchor410 can be similar in design to the anchoring device described in co-pending U. S. Provisional Application Serial Numbers 60/528, 084, entitled "Bariatric Sleeve", filed December 9,2003, and 60/544,527, entitled "Methods And Apparatus For Using A Sleeve Within The Gastrointestinal Tract", filed February 13,2004, (for both see US-A-2005/0125020).

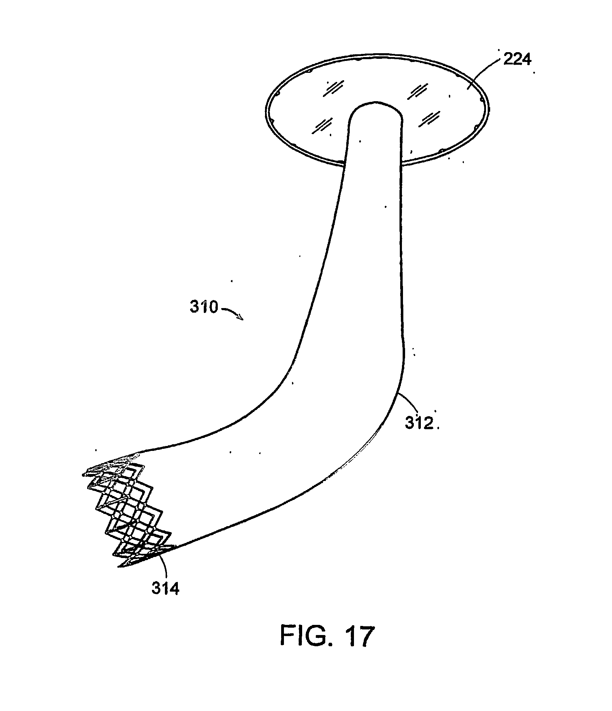

FIG. 17 shows the stomach portion of a two-piece system 310. The two piece system 310 includes a stomach portion 312 and an intestine, or lower portion. The stomach portion 312 includes a restrictive membrane224, upper sleeve 302 and connector assembly 314. The stomach portion 224, 312,314 is collapsed and introduced into the stomach 102 after the lower portion which includes a lower sleeve with a pyloric stent, described in more detail below, and an anchoring ring have been anchored in the pylorus 305.

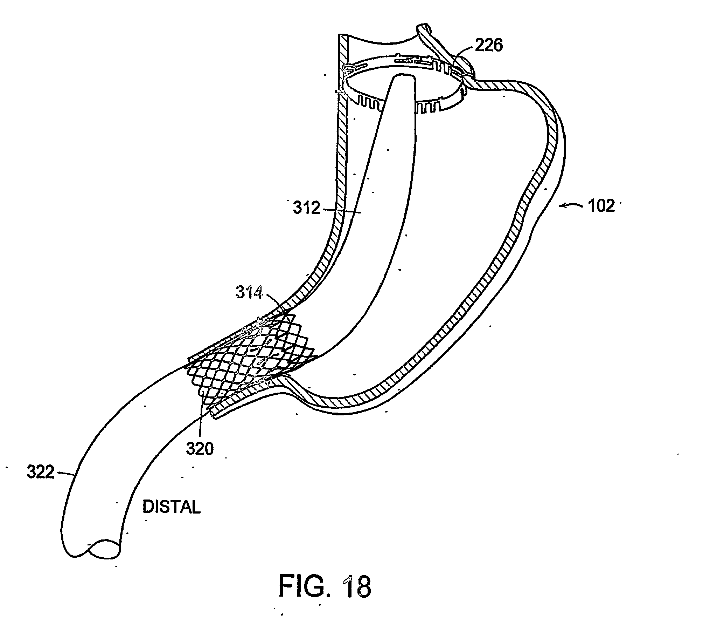

FIG. 18 shows the two-piece sleeve system 310 inserted into the gastrointestinal tract of an animal. The stomach sleeve 312 with the connector assembly 314 is coupled to an anchor, or stent 320, which is, in turn attached to the lower (intestine) sleeve 322. The upper sleeve312 is attached, or bonded to the restrictive membrane 224, as described above. Thus, in some embodiments, the upper sleeve 312 and restrictive membrane 224 are not detachable. With the anchor ring 226 anchored in the stomach 102 and the lower sleeve 322 with the expanded anchor 320 with anchors deployed in the inside wall of the pylorus 305, the upper sleeve 312, restrictive membrane 224 and connector assembly 314 are collapsed and introduced into the stomach 102.

The barbs grip onto the muscle to anchor the implant device 216 in place so that the implant device 216 can not be dragged into the stomach 102 or down into the intestines with movement of the stomach 102 and the intestines. The anchor 314 is non-woven, collapsible and self-expanding, allowing endoscopic insertion and removal of the implant device. The stent 314 includes a plurality of flat struts forming an open space pattern to ease collapsing while ensuring self-expansion. The open space pattern allows for collapsing into a catheter for endoscopic delivery and removal. The struts may be manufactured from heat-treated spring steel, or an alloy such as Nitinol or MP35N. The stent can be formed from a tube of material by laser cutting followed by expansion and heat setting, or other methods well known to those skilled in the art.

In an alternate embodiment the struts can be formed separately and the strut intersections can be welded or attached by other means well known to those skilled in the art. Visually the struts form sections around the circumference of the stent. Each section has a series of triangles with each triangle defined by one distal strut connection and two proximal strut connections. The ratio of the collapsed diameter to the expanded diameter of the stent is roughly about 1: 4.

The ends of the struts at the proximal end of the stent are elongated and shaped to provide barbs to anchor to the muscle in the pyloric portion of the stomach. Pairs of barbs at the proximal end of the stent are elongated and can be shaped to provide opposed barbs to anchor the stent in the muscle of the pylorus. The strut ends protrude outward from the outer surface of the stent in opposite directions. They may be perpendicular to each other. The barbs at the ends of the respective opposed strut ends dig into the pylorus muscle to anchor the stent. The barbs at the end of the protruding opposed strut ends prevent movement of the stent in either direction; that is, they prevent movement of the stent into the stomach and prevent movement of the stent down through the duodenum.

The distal end of the collapsed stomach sleeve connector is placed inside the duodenal sleeve anchoring pyloric stent. Endoscopic graspers are used to lock the connector to the pyloric stent. Lastly, the restrictive drum is coupled to the stomach anchor ring.

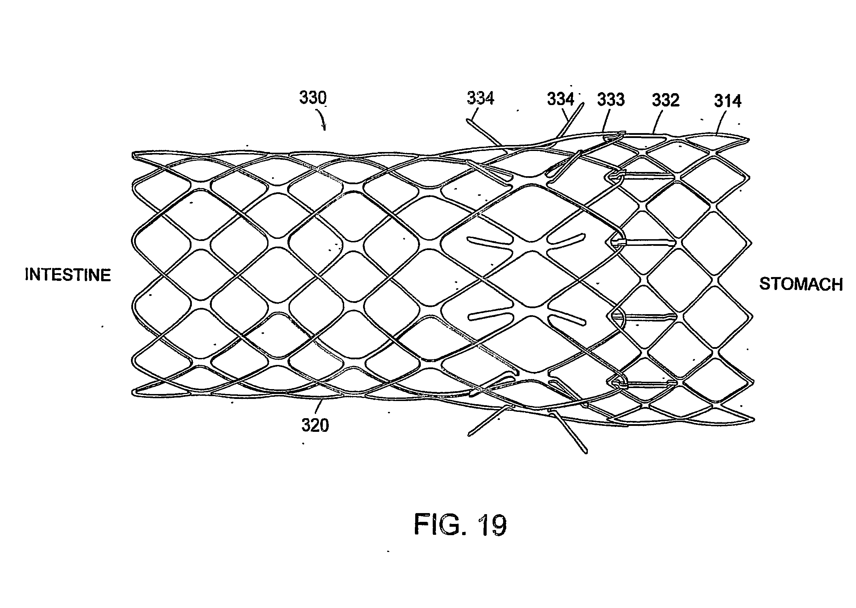

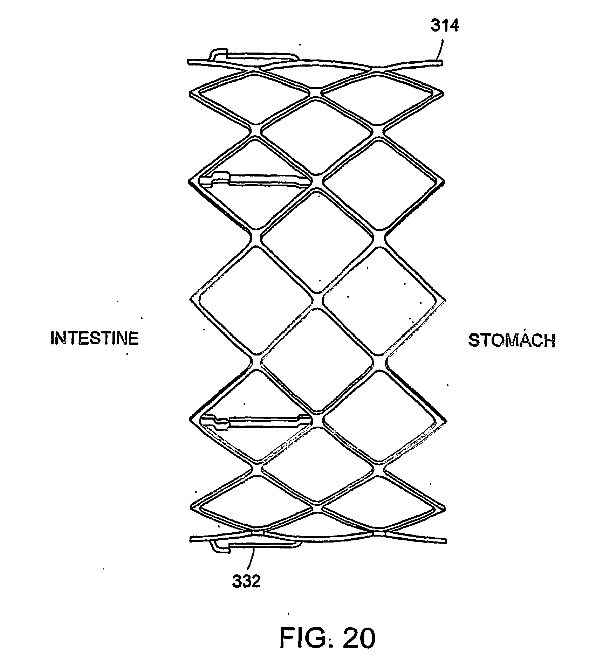

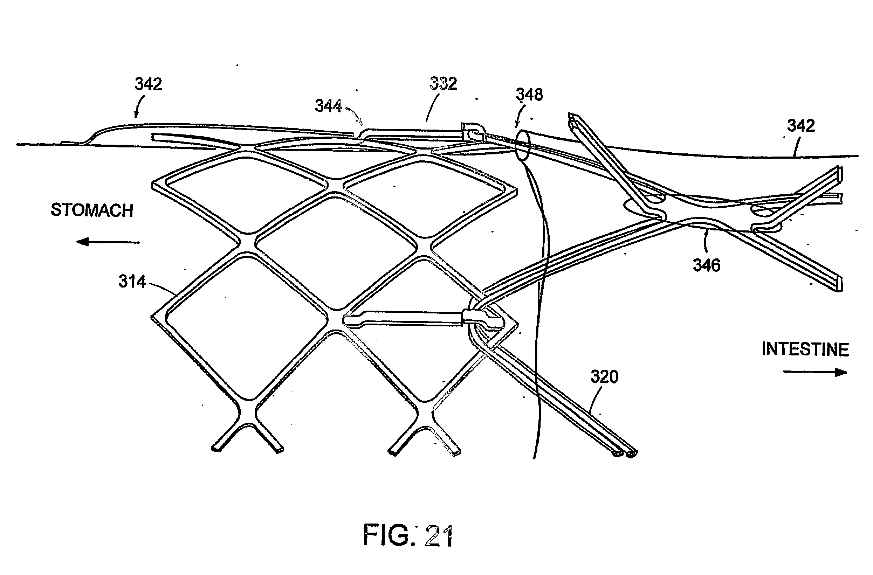

Figs. 19 and 20 illustrate the anchor 320 and 314. The anchor 320 is covered by the sleeve as described in co-pending U. S. Utility Patent Application No. 10/339,786 (see US-A-7025791) but the anchor 320 is shown with the sleeve removed for illustrative purposes. FIG. 19 shows the anchor 320 and connector 314 after they have been fastened, or locked together. FIG. 20 shows the connector 314 with locking details 322. The anchor 320 and connector 314 are typically both covered by a sleeve. Apertures are provided in the sleeve such that the connectors and barbs extend through the sleeve. In some embodiments, the sleeve can be bonded to the stent to minimize the exposed metal. The portions are coupled by hooking the exposed portion of the pylorus stent to the connectors located on the proximal stomach sleeve anchor. FIG. 21 illustrates another locking means in which the connector is coupled to the stent in both radial and longitudinal directions.

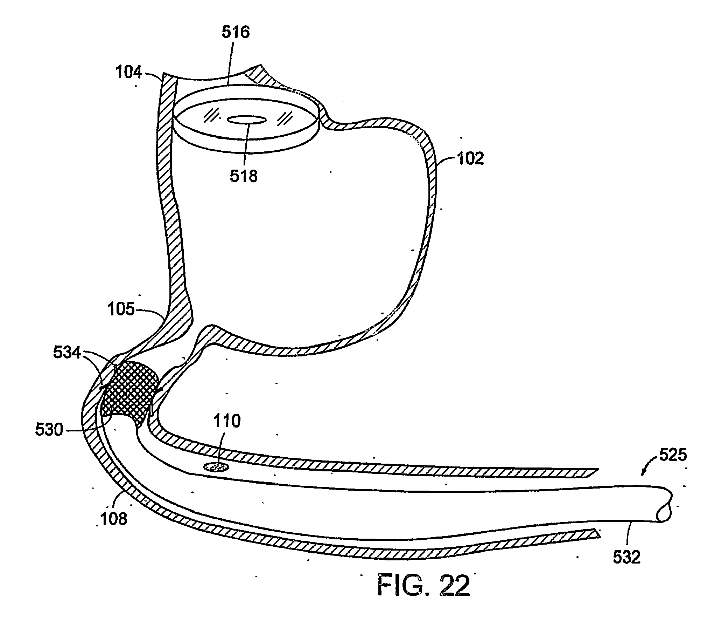

FIG. 22 illustrates an alternative embodiment of a gastrointestinal implant including a restrictive device 518, and an elongated tube 525, or sleeve. This configuration is similar to the two-piece system shown in FIG. 17, except that the sleeve is not attached to the restrictive device. The elongated tube 525 generally includes a flexible sleeve 532, similar to the flexible sleeves described above, attached at one end to an anchor 530. The anchor 530 can also be similar to the anchors described above, being tubular and secured to the gastrointestinal tract 110, by any of the attachment means described above, including barbs 534. For example, the elongated tube 525 can be anchored at its proximal end to the intestine, below the pylorus 305, as illustrated.

While this invention has been particularly shown and described with references to preferred embodiments thereof, it will be understood by those skilled in the art that various changes in form and details may be made therein without departing from the scope of the invention encompassed by the appended claims.

## Claims

1. A gastrointestinal implant device having an anchor (226) and a restrictive member (224) removably coupled to the anchor, **characterised by**
a planar restrictive member (224), having an aperture (218) therein, configured for implantation into a stomach (102) of an animal, the restrictive member (224) configured to divide the inner volume of the stomach (102) into a proximal chamber (200) and a distal chamber (222), and the restrictive member (234) having an exterior perimeter (217) adapted to contact inner walls of the stomach (102), and the restrictive member (224) having
an anchor (226) configured to be fixedly coupled to the stomach for securing the restrictive member (224) within the stomach.

2. The gastrointestinal implant device of claim 1, wherein the restrictive member (224) is a membrane having the exterior perimeter (217) and defining an interior aperture (218).

3. The gastrointestinal implant device of claim 2, wherein the aperture (218) is substantially circular, having a diameter between 1 and 5 centimeters.

4. The gastrointestinal implant device of claim 2, wherein the restrictive member (224) is substantially circular, having an outer diameter between 7 and 20 centimeters.

5. The gastrointestinal implant device of claim 1, wherein the restrictive member (224) is both flexible, allowing the restrictive member (224) to deform, and non- elastic, ensuring that the surface area of the restrictive member (224) does not vary substantially.

6. The gastrointestinal implant device of claim 5, wherein the restrictive member (224) is substantially non-permeable.

7. The gastrointestinal implant device of claim 6, wherein the non-permeable restrictive member (224) comprises a permeable material impregnated with an impermeable coating.

8. The gastrointestinal implant device of claim 7, wherein the permeable material is selected from the group consisting of: natural fibers; synthetic fibers; polyester fibers; and combinations thereof, and the impermeable coating is selected from the group consisting of silicone; urethane; and combinations thereof.

9. The gastrointestinal implant device of claim 5, wherein the restrictive member (224) is formed from a composite material prepared using an otherwise elastic material together with a matrix of fibers impregnated therein, the resulting composite being substantially non-elastic.

10. The gastrointestinal implant device of claim 1, wherein the restrictive member (224) comprises a feature configured for coupling to the anchor (226).

11. The gastrointestinal implant device of claim 10, wherein the feature comprises a loop.

12. The gastrointestinal implant device of claim 1, wherein the anchor (226) is fixedly coupled to the stomach and the restrictive member (224) is removably coupled to the anchor (226), allowing the restrictive member (224) to be removed and replaced as necessary, without having to remove the anchor (226).

13. The gastrointestinal implant device of claim 1, wherein the anchor (226) includes a plurality of retractable spring clips (238), each spring clip (238) configured to penetrate the muscular tissue of the stomach.

14. The gastrointestinal implant device of claim 13, wherein the anchor (226) includes a spring clip-retaining device (226) configured to retain the spring clips (238) in a non-deployed position during insertion and/or removal of the anchor (226).

15. The gastrointestinal implant device of claim 14, wherein the spring clip-retaining device (226) comprises a retaining ring removably coupled to the anchor (226) and engaging the plurality of spring clips (238) securing the plurality of spring clips (238) in a non-deployed position.

16. The gastrointestinal implant device of claim 15, wherein the spring-clip- retaining device (226) comprises a retaining aperture (234) defined by the anchor (226) itself.

17. The gastrointestinal implant device of claim 1, wherein the anchor (304) is comprised of a shape-memory material.

18. The gastrointestinal implant device of claim 17, wherein the shape-memory material comprises a nickel-titanium (Ni-Ti) alloy.

19. The gastrointestinal implant device of claim 1, wherein the anchor (226) comprises a feature (230) configured for coupling to the restrictive member (224).

20. The gastrointestinal implant device of claim 19, wherein the feature comprises a hook (230).

## Patentansprüche

1. Magen-Darm-Implantatanordnung, aufweisend eine Verankerung (226) und ein Beschränkungselement (224), welches abnehmbar mit der Verankerung gekoppelt ist,
**gekennzeichnet durch**
ein flächiges Beschränkungselement (224), aufweisend eine Öffnung (218), ausgelegt zur Implantation in einem Magen (102) eines Tieres, wobei das Beschränkungselement (224) derart gestaltet ist, um das innere Volumen des Magens (102) zu teilen in eine obere Kammer (200) und eine untere Kammer (222), und wobei das Beschränkungselement (234) einen äußeren Umriss (217) aufweist, der ausgelegt ist um die inneren Wände des Magens (102) zu kontaktieren und das Beschränkungselement (224) weist eine Verankerung (226) auf, zur festen Kopplung mit dem Magen um das Beschränkungselement (224) innerhalb des Magens zu sichern.

2. Magen-Darm-Implantatanordnung nach Anspruch 1, worin das Beschränkungselement (224) eine Membran ist mit einem äußeren Umriss (217) und mit einer inneren Öffnung (218).

3. Magen-Darm-Implantatanordnung nach Anspruch 2, worin die Öffnung (218) im Wesentlichen ringförmig ist und einen Durchmesser zwischen 1 und 5 Zentimeter aufweist.

4. Magen-Darm-Implantatanordnung nach Anspruch 2, worin das Beschränkungselement (224) im Wesentlichen ringförmig ist und einen äußeren Durchmesser zwischen 7 und 20 Zentimeter aufweist.

5. Magen-Darm-Implantatanordnung nach Anspruch 1, worin das Beschränkungselement (224) sowohl flexibel ist, um Deformationen des Beschränkungselements (224) zu ermöglichen, als auch nicht-elastisch, um sicherzustellen, dass der Oberflächenbereich des Beschränkungselements (224) im Wesentlichen nicht variiert.

6. Magen-Darm-Implantatanordnung nach Anspruch 5, worin das Beschränkungselement (224) im Wesentlichen undurchlässig ist.

7. Magen-Darm-Implantatanordnung nach Anspruch 6, worin das undurchlässige Beschränkungselement (224) ein durchlässiges Material umfasst, welches mit einer undurchlässigen Beschichtung imprägniert ist.

8. Magen-Darm-Implantatanordnung nach Anspruch 7, worin das durchlässige Material ausgewählt ist aus der Gruppe bestehend aus: natürliche Fasern; synthetische Fasern; Polyesterfasern; und einer Kombination daraus und dass die imprägnierende Beschichtung ausgewählt ist aus der Gruppe bestehend aus: Silikon; Urethan; und Kombinationen daraus.

9. Magen-Darm-Implantatanordnung nach Anspruch 5, worin das Beschränkungselement (224) dargestellt ist aus einem zusammengesetzten Material, welches präpariert ist unter Verwendung eines anderen elastischen Materials zusammen mit einer Matrix aus Fasern, die darin imprägniert sind, wobei die sich ergebende Zusammensetzung im Wesentlich nicht elastisch ist.

10. Magen-Darm-Implantatanordnung nach Anspruch 1, worin das Beschränkungselement (224) ein Merkmal umfasst, welches zur Kopplung mit der Verankerung (226) ausgelegt ist.

11. Magen-Darm-Implantatanordnung nach Anspruch 10, worin das Merkmal eine Schlinge umfasst.

12. Magen-Darm-Implantatanordnung nach Anspruch 1, worin die Verankerung (226) fest mit dem Magen verbunden ist und das Beschränkungselement (224) abnehmbar mit dem Magen (226) gekoppelt ist um eine Ablösung des Beschränkungselements (224) zu ermöglichen, so dass dieses falls notwendig ersetzbar ist, ohne dass die Verankerung (226) zu entfernen ist.

13. Magen-Darm-Implantatanordnung nach Anspruch 1, worin die Verankerung (226) eine Vielzahl von zurückziehbaren Federklammern (238) umfasst, wobei jede Federklammer (238) zum Durchdringen des Muskelgewebes des Magens ausgelegt ist.

14. Magen-Darm-Implantatanordnung nach Anspruch 13, worin die Verankerung (226) eine Federklammer-Verankerungsvorrichtung (226), ausgelegt zum Zurückhalten der Federklammern (238) in einer nicht betätigten Position, während der Einführung und/oder während der Entfernung von der Verankerung (226).

15. Magen-Darm-Implantatanordnung nach Anspruch 14, worin die Federklammer-Verankerungsvorrichtung (226) einen Rückhaltering umfasst, welcher abnehmbar mit der Verankerung (226) gekoppelt ist und zusammenwirkt mit der Vielzahl von Federklammern (238), wobei dieser die Vielzahl der Federklammern (238) in einer nicht betätigten Position sichert.

16. Magen-Darm-Implantatanordnung nach Anspruch 15, worin die Federklammer-Verankerungsvorrichtung (226) eine Rückhalteöffnung (234) aufweist, welche durch die Verankerung (226) bestimmt ist.

17. Magen-Darm-Implantatanordnung nach Anspruch 1, worin die Verankerung (304) aus einem Form-Gedächtnis-Material aufweist.

18. Magen-Darm-Implantatanordnung nach Anspruch 17, worin das Form-Gedächtnis-Material eine Nickel Titan (Ni-Ti) Legierung aufweist.

19. Magen-Darm-Implantatanordnung nach Anspruch 1, worin die Verankerung (226) ein Merkmal (230) umfasst, ausgelegt zur Kopplung mit dem Rückhalteelement (224).

20. Magen-Darm-Implantatanordnung nach Anspruch 19, worin das Merkmal eine Öse (230) umfasst.

## Revendications

1. Implant gastro-intestinal comportant une ancre (226) et un élément de restriction (224) ; **caractérisé par** :
un élément de restriction plan (224) comportant une ouverture (218), agencé pour être implanté dans l'estomac (102) d'un animal, l'élément de restriction (224) étant agencé pour diviser le volume interne de l'estomac (102) en une chambre proximale (200) et une chambre distale (222), et l'élément de restriction (234) ayant une périphérie extérieure (217) apte à contacter des parois internes de l'estomac (102) ; et l'élément de restriction (224) ayant une ancre (226) agencée pour être couplée de façon fixe à l'estomac pour fixer l'élément de restriction (224) dans l'estomac.

2. Implant gastro-intestinal selon la revendication 1, dans lequel l'élément de restriction (224) est une membrane ayant ladite périphérie extérieure (217) et définissant une ouverture intérieure (218).

3. Implant gastro-intestinal selon la revendication 2, dans lequel l'ouverture (218) est sensiblement circulaire, ayant un diamètre compris entre 1 et 5 centimètres.

4. Implant gastro-intestinal selon la revendication 2, dans lequel l'élément de restriction (224) est sensiblement circulaire, ayant un diamètre extérieur compris entre 7 et 20 centimètres.

5. Implant gastro-intestinal selon la revendication 1, dans lequel l'élément de restriction (224) est à la fois flexible, ce qui permet une déformation de l'élément de restriction (224), et non élastique, ce qui assure que la surface de l'élément de restriction (224) ne varie pas notablement.

6. Implant gastro-intestinal selon la revendication 5, dans lequel l'élément de restriction (224) est sensiblement non perméable.

7. Implant gastro-intestinal selon la revendication 6, dans lequel l'élément de restriction non perméable (224) comprend un matériau perméable imprégné d'un revêtement imperméable.

8. Implant gastro-intestinal selon la revendication 7, dans lequel le matériau perméable est choisi dans le groupe comprenant : des fibres naturelles ; des fibres synthétiques ; des fibres de polyester ; et leurs combinaisons, et le revêtement imperméable est choisi dans le groupe comprenant le silicone, l'uréthane, et leurs combinaisons.

9. Implant gastro-intestinal selon la revendication 5, dans lequel l'élément de restriction (224) est formé en un matériau composite préparé en utilisant un matériau par lui-même élastique conjointement avec une matrice de fibres imprégnée à l'intérieur, le composite résultant étant sensiblement non élastique.

10. Implant gastro-intestinal selon la revendication 1, dans lequel l'élément de restriction (224) comprend un élément caractéristique agencé pour un couplage à l'ancre (226).

11. Implant gastro-intestinal selon la revendication 10, dans lequel ledit élément caractéristique comprend une boucle.

12. Implant gastro-intestinal selon la revendication 1, dans lequel l'ancre (226) est couplée de façon fixe à l'estomac et l'élément de restriction (224) est couplé de façon amovible à l'ancre (226), ce qui permet à l'élément de restriction (224) d'être retiré et remplacé si nécessaire, sans devoir retirer l'ancre (226).

13. Implant gastro-intestinal selon la revendication 1, dans lequel l'ancre (226) comprend une pluralité de brides à ressort rétractables (238), chaque bride à ressort (238) étant agencée pour pénétrer dans le tissu musculaire de l'estomac.

14. Implant gastro-intestinal selon la revendication 13, dans lequel l'ancre (226) comprend un dispositif de maintien de brides à ressort (226) agencé pour retenir les brides à ressort (238) dans une position non déployée pendant l'insertion et/ou le retrait de l'ancre (226).

15. Implant gastro-intestinal selon la revendication 14, dans lequel le dispositif de maintien de brides à ressort (226) comprend un anneau de maintien couplé de façon amovible à l'ancre (226) et en contact avec la pluralité de brides à ressort (238), fixant la pluralité de brides à ressort (238) dans une position non déployée.

16. Implant gastro-intestinal selon la revendication 15, dans lequel le dispositif de maintien de brides à ressort (226) comprend une ouverture de maintien (234) définie par l'ancre (226) elle-même.

17. Implant gastro-intestinal selon la revendication 1, dans lequel l'ancre (304) est constituée d'un matériau à mémoire de forme.

18. Implant gastro-intestinal selon la revendication 17, dans lequel le matériau à mémoire de forme comprend un alliage nickel-titane (Ni-Ti).

19. Implant gastro-intestinal selon la revendication 1, dans lequel l'ancre (226) comprend un élément caractéristique (230) agencé pour un couplage à l'élément de restriction (224).

20. Implant gastro-intestinal selon la revendication 19, dans lequel ledit élément caractéristique comprend un crochet (230).
